# EUROPEAN PATENT SPECIFICATION

(11) **EP 2 623 047 B1**
(45) Date of publication and mention of the grant of the patent: **09.05.2018**
(21) Application number: 11828031.2
(22) Date of filing: 24.08.2011
(51) Int. Cl.: A61B 17/16

(54) **CRANIOTOMY DRILL**
KRANIEKTOMIEBOHRER
FORET POUR CRANIOTOMIE

(30) Priority: 30.09.2010 CN 201010298084
(43) Date of publication of application: 07.08.2013
(73) Proprietor: CHONGQING RUZER PHARMACEUTICAL CO., LTD., Yubei District Chongqing 401120 (CN)
(72) Inventor: YE, Lei, Chongqing 401120 (CN); ZHOU, Jian, Chongqing 401120 (CN); FENG, Hua, Chongqing 401120 (CN); LI, Fei, Chongqing 401120 (CN); ZHU, Hengyang, Chongqing 401120 (CN); LI, Congxiao, Chongqing 401120 (CN)
(74) Representative: Algemeen Octrooi- en Merkenbureau B.V.
(86) International application number: PCT/CN2011/078827
(87) International publication number: WO 2012/041135

(56) References cited:
- CN-A- 1 931 103
- CN-A- 101 045 006
- CN-A- 101 926 670
- CN-U- 201 798 778
- CN-U- 201 822 887
- CN-Y- 2 855 334
- CN-Y- 201 198 128
- DE-A1- 19 619 774
- US-A- 5 591 170

## Description

### Field of the Invention

The present invention relates to a bone drill motor for use in surgery.

### Description of the Prior Art

In surgery, a cranial drill is a tool commonly used for craniotomy. The cranial drill comprises a main machine, a reducer, a locking seat and a drill head. The main machine comprises a handle in the lower portion, and a cylindrical housing of a containing part and an electric motor in shape of a cylinder both in the upper part. During assembly, the electric motor needs to be inserted into the cavity of the cylindrical containing part in the upper part of the housing, and then the electric motor is fixed with tightening screws. However, the volume of the upper portion of the housing becomes larger as a result of this structure, increasing the overall weight of the cranial drill, and additionally the tightening screw may come loose over time and the electric motor would fall out from the containing cavity to potentially cause accident during surgery.

### Summary of the Invention

The object of the present invention is to provide a cranial drill with small volume and light weight.

The object of the present invention is accomplished by adopting the following scheme.

A cranial drill comprises a main machine, a reducer and a locking seat that are successively threadedly connected, having a drill transmission rod interconnected to the output shaft of the reducer inserted in the locking seat, characterized in that, the main machine comprises a handle in the lower portion, and a containing part in the upper portion, the containing part having a stator and rotor of a DC electric motor provided therein, the wall of the containing part defining the housing of the stator of the DC electric motor. According to the invention, in order to facilitate the mounting and unmounting of the drill transmission rod, the locking seat comprises a flange with a through hole, the inner wall of the small cylinder on the upside of the flange is arranged with bearings and supporting members that support the bearings. The flange is also provided with a locking sleeve which is arranged with a first protrusion on the inner wall of the upper portion thereof and a second protrusion on the inner wall of the lower portion thereof. A spring is arranged between the second protrusion and the upper bottom face of the flange. A pressing plate is threadedly connected with an outer wall of the top portion of the small cylinder on the upside of the flange and presses against the first protrusion. Holes of various sizes are provided on the inner wall of the small cylinder where corresponds to the second protrusion and on the supporting members to form stepped holes which are provided with steel balls therein.

In order to transfer uniform force to the transmission rod, the bearings can be two which are respectively positioned below the outer wall and above the upper bottom face.

In order to ensure the bearings and the pressing member are secured, the upper bottom face is arranged with a pressing member threadedly connected with the inner wall of the upper bottom face for tightly abutting the bearings and the supporting member.

In order to ensure smooth movement of the transmission rod, the bearings can be two which are respectively positioned below the outer wall and above the pressing member.

In order to facilitate connecting the reducer, the inner wall of the big cylinder on the lower side of the flange is provided with inner threads. According to the invention, in order to facilitate leading out the wires from the stator unitedly, the rear portion of the containing part is arranged with a blocking plate with holes, the end cover is threadedly connected with the rear end of the containing part in tight fitting. The wires of the stator of the DC electric motor go through the holes on the block plate and enter the inside of the handle.

In order to waterproof, all wires of the stator of the DC electric motor have waterproof layer arranged thereon.

In order to strengthen the seal on the reducer, the output shaft of the reducer is provided with lip-shaped sealing ring.

The present invention is simple in structure, having the wall of the containing part defining the housing of the stator of the electric motor, thus the volume and the whole weight are reduced. It is safe in operation, without the risk of the electric motor falling out. And its waterproof performance is good, by utilizing materials and processes of waterproof technology and an integrative design. When the transmission rod is to be inserted, one only has to press down the locking sleeve, then the second protrusion moves away from the steel ball which engages the rod body of the transmission rod and rolls toward outside. When the transmission rod reaches the designated position, one releases the locking sleeve which moves up upon the effect of the spring, the second protrusion engages the steel ball and moves inwardly to snap into the snap-slot on the transmission rod which is thus secured. In such a locking manner, the locking and loosening operations are easy and convenient to apply, providing a firm securing effect when in locking status and improved safety.

### Brief Description of the Drawings

Figure 1 is a structural schematic view of a main machine in an embodiment of the present invention;
Figure 2 is a right view of Figure 1;
Figure 3 is a view of Figure 1 taken along line A-A;
Figure 4 is a structural schematic view of a locking seat in an embodiment of the present invention;
Figure 5 is an overall structural schematic view of an embodiment of the present invention.

### Detailed Description of the Preferred Embodiments

Hereinafter further description will be made by incorporating figures and embodiments to illustrate the present invention.

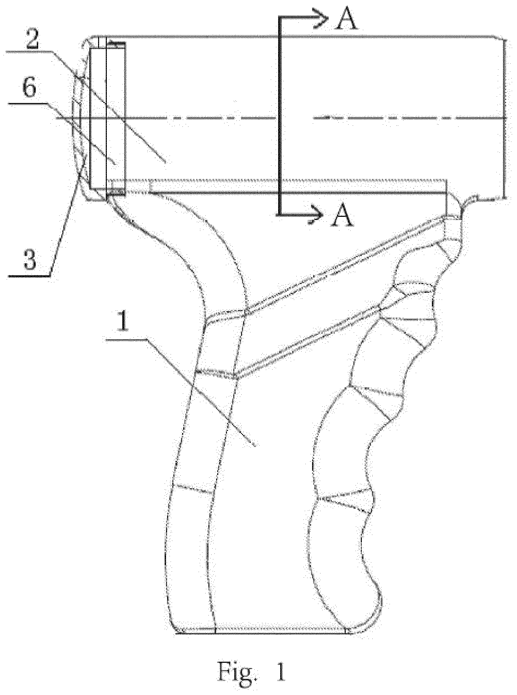

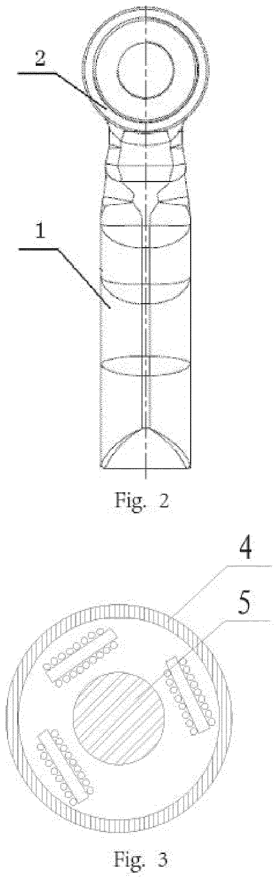

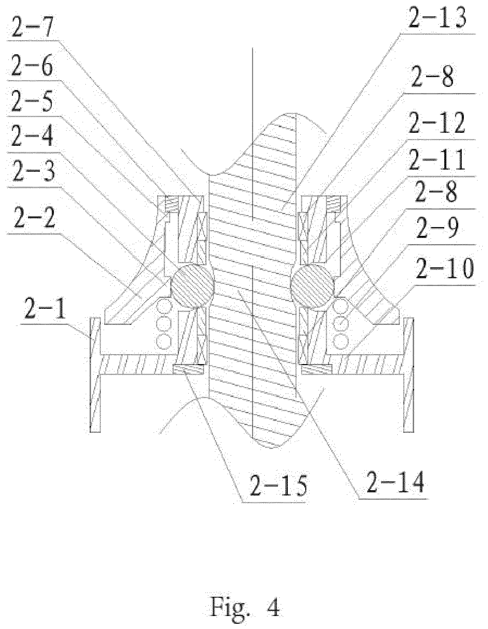

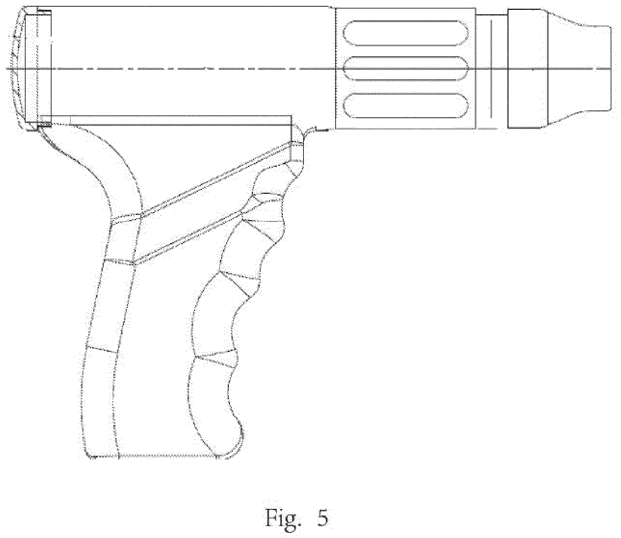

As can be seen from Figure 1 to Figure 5:
A cranial drill, comprises a main machine, a reducer and a locking seat that are successively threadedly connected, having a turning transmission rod interconnected to the output shaft of the reducer inserted in the locking seat, the main machine comprises a handle 1 in the lower portion, and a containing part 2 in the upper portion, the containing part 2 having a stator and rotor 5 of a DC electric motor provided therein, the wall 4 of the containing part 2 defining the housing of the stator of the DC electric motor. A block plate 6 with holes is arranged in the rear portion of the containing part 2, dividing which into two cavities with the front cavity arranged with a stator of the electric motor, end cover 3 is threadedly connected with the rear end of the containing part 2 in tight fitting. The wires of the stator of the DC electric motor go through the holes on the block plate 6, enter the inside of the handle 1 after passing through the rear cavity. All wires of the stator of the DC electric motor have waterproof layer arranged thereon.

The locking seat comprises a flange 2-1 with a through hole. Two bearings 2-8 are arranged on the inner wall of the small cylinder on the upside of the flange, respectively positioned below the outer wall 2-7 and above a pressing member 2-15, and between which a supporting member 2-12 is arranged. An upper bottom face 2-10 is arranged with a pressing member 2-15 threadedly connected with the inner wall of the upper bottom face for tightly abutting the bearings 2-8 and the supporting member 2-12. The two bearings can effectively ensures smooth rotation of the transmission rod to produce lesser heat, while the existence of the supporting member 2-12 adds to the firmness between the bearings to reduce the likelihood of detachment.

The flange is also provided with a locking sleeve 2-2 having a first protrusion 2-5 provided on the inner wall of the upper portion thereof and a second protrusion 2-3 provided on the inner wall of the lower portion thereof. A spring 2-9 is arranged between the second protrusion 2-3 and the upper bottom face 2-10 of the flange. A pressing plate 2-6 is threadedly connected with an outer wall 2-7 of the top portion of the small cylinder on the upside of the flange and presses against the first protrusion 2-5. Holes of various sizes are provided on the inner wall of the small cylinder where corresponds to the second protrusion 2-3 and on the supporting member 2-12 to form stepped holes 2-11 which are provided with steel balls 2-4 therein. The diameter of the hole on the inner wall of the small cylinder is greater than that of the steel ball 2-4, and the diameter of the hole on the supporting member 2-12 is less than that of the steel ball 2-4.

A transmission rod 2-13 is provided with a groove 2-14 in the corresponding position to the stepped holes 2-11. The inner wall of the big cylinder on the lower side of the flange 2-1 is provided with inner threads. In this embodiment, the number of stepped holes 2-11 and steel balls 2-4 are both counted to be two. As a variant, the number of steel balls and stepped holes may be set to be three, so that an improved locking effect is provided.

The techniques described herein are exemplary, and should not be construed as implying any particular limitation on the present disclosure. It should be understood that various alternatives, combinations and modifications could be devised by those skilled in the art without departing from the technical scheme content of the present invention. The present disclosure is intended to embrace all alternatives, modifications and variances that fall within the scope of the appended claims.

## Claims

1. A cranial drill, comprising a main machine, a reducer and a locking seat that are successively threadedly connected, having a drill transmission rod interconnected to an output shaft of the reducer inserted in the locking seat, wherein the main machine comprises a handle (1) in the lower portion, and a containing part (2) in the upper portion, the containing part (2) having a stator and rotor (5) of a DC electric motor provided therein, a wall (4) of the containing part (2) defining a housing of the stator of the DC electric motor;
wherein the rear portion of the containing part (2) is arranged with a blocking plate (6) with holes, an end cover (3) is threadedly connected with the rear end of the containing part (2) in tight fitting; wires of the stator of the DC electric motor go through the holes on the block plate (6) and enter the inside of the handle (1);
wherein the locking seat comprises: a flange (2-1) with a through hole, an inner wall of a small cylinder on the upside of the flange arranged with bearings (2-8) and supporting members (2-12) that support the bearings (2-8), the flange also provided with a locking sleeve (2-2) which is arranged with a first protrusion (2-5) on the inner wall of the upper portion thereof and a second protrusion (2-3) on the inner wall of the lower portion thereof; a spring (2-9) is arranged between the second protrusion (2-3) and the upper bottom face (2-10) of the flange; a pressing plate (2-6) is threadedly connected with an outer wall (2-7) of a top portion of the small cylinder on the upside of the flange and presses against the first protrusion (2-5); holes of various sizes are provided on the inner wall of the small cylinder corresponding to the second protrusion (2-3) and on the supporting members (2-12) to form stepped holes (2-11) which are provided with steel balls (2-4) therein.

2. The cranial drill according to claim 1, **characterized in that**, the upper bottom face (2-10) is arranged with a pressing member (2-15) threadedly connected with the inner wall of the upper bottom face for tightly abutting the bearings (2-8) and the supporting member (2-12).

3. The cranial drill according to claim 2, **characterized in that**, the bearings (2-8) are numbered to be two which are respectively positioned below the outer wall (2-7) and above the pressing members (2-15).

4. The cranial drill according to claim 3, **characterized in that**, the inner wall of the big cylinder on the lower side of the flange (2-1) is provided with inner threads.

5. The cranial drill according to claim 4, **characterized in that**, all wires inside the stator of the DC electric motor have waterproof layer arranged thereon.

6. The cranial drill according to claim 5, **characterized in that**, the output shaft of the reducer is provided with a lip-shaped sealing ring.

## Patentansprüche

1. Kranialbohrer, umfassend einen Hauptapparat, eine Reduzierung und einen Arretierungssitz, die nacheinander durch ein Gewinde verbunden werden, mit einer Bohrübertragungsstange, die mit einer Ausgabewelle der in den Arretierungssitz eingefügten Reduzierung verbunden ist, wobei der Hauptapparat einen Griff (1) in dem unteren Abschnitt und einen aufnehmenden Teil (2) in dem oberen Abschnitt umfasst, wobei der aufnehmende Teil (2) einen Stator und einen Rotor (5) eines darin vorgesehenen Gleichstromelektromotors aufweist und wobei eine Wand (4) des aufnehmenden Teils (2) ein Gehäuse des Stators des Gleichstromelektromotors definiert;
wobei der hintere Abschnitt des aufnehmenden Teils (2) mit einer Blockierscheibe (6) mit Bohrungen eingerichtet ist, wobei eine Endabdeckung (3) durch ein Gewinde mit dem hinteren Ende des aufnehmenden Teils (2) eng sitzend verbunden ist, wobei Leitungen des Stators des Gleichstromelektromotors durch die Bohrungen an der Blockierscheibe (6) gehen und in das Innere des Griffes (1) eintreten;
wobei der Arretierungssitz Folgendes umfasst: einen Flansch (2-1) mit einer Durchgangsbohrung, eine innere Wand eines kleinen Zylinders an der Oberseite des Flansches, der mit Lagern (2-8) und tragenden Elementen (2-12), die die Lager (2-8) tragen, eingerichtet ist, wobei der Flansch auch mit einer Arretierungshülse (2-2) versehen ist, die mit einem ersten Vorsprung (2-5) an der inneren Wand des oberen Abschnitts von dieser und einem zweiten Vorsprung (2-3) an der inneren Wand des unteren Abschnitts von dieser eingerichtet ist; eine Feder (2-9) ist zwischen dem zweiten Vorsprung (2-3) und der oberen Unterseite (2-10) des Flansches angeordnet; eine Pressscheibe (2-6) ist durch ein Gewinde mit einer äußeren Wand (2-7) eines oberen Abschnitts des kleinen Zylinders an der Oberseite des Flansches verbunden und drückt gegen den ersten Vorsprung (2-5); Bohrungen verschiedener Größe sind an der inneren Wand des kleinen Zylinders korrespondierend zu dem zweiten Vorsprung (2-3) und an den tragenden Elementen (2-12) vorgesehen, um Stufenbohrungen (2-11) zu bilden, die mit Stahlkugeln (2-4) in diesen versehen sind.

2. Kranialbohrer nach Anspruch 1,
**dadurch gekennzeichnet, dass**
die obere Unterseite (2-10) mit einem Presselement (2-15) eingerichtet ist, das durch ein Gewinde mit der inneren Wand der oberen Unterseite zum engen Anliegen der Lager (2-8) und der tragenden Elemente (2-12) verbunden ist.

3. Kranialbohrer nach Anspruch 2,
**dadurch gekennzeichnet, dass**
die Lager (2-8) gezählt sind, um zwei zu sein, die jeweils unter der äußeren Wand (2-7) und über den Presselementen (2-15) positioniert sind.

4. Kranialbohrer nach Anspruch 3,
**dadurch gekennzeichnet, dass**
die innere Wand des großen Zylinders an der unteren Seite des Flansches (2-1) mit Innengewinden versehen ist.

5. Kranialbohrer nach Anspruch 4,
**dadurch gekennzeichnet, dass**
auf allen Leitungen innerhalb des Stators des Gleichstromelektromotors wasserdichte Schichten angeordnet sind.

6. Kranialbohrer nach Anspruch 5,
**dadurch gekennzeichnet, dass**
die Abtriebswelle der Reduzierung mit einem lippenförmigen Dichtring versehen ist.

## Revendications

1. Trépan crânien, comprenant une machine principale, un réducteur et un siège de verrouillage qui sont reliés successivement par filetage, ayant une tige de transmission de trépan reliée à un arbre de sortie du réducteur inséré dans le siège de verrouillage, où la machine principale comprend une poignée (1) dans la partie inférieure, et une partie de réception (2) dans la partie supérieure, la partie de réception (2) ayant un stator et un rotor (5) d'un moteur électrique à courant continu prévu à l'intérieur de celle-ci, une paroi (4) de la partie de réception (2) définissant un boîtier du stator du moteur électrique à courant continu ;
dans lequel la partie arrière de la partie de réception (2) est agencée avec une plaque de blocage (6) ayant des trous, un couvercle d'extrémité (3) est relié par filetage à l'extrémité arrière de la partie de réception (2) de manière ajustée ; des fils du stator du moteur électrique à courant continu traversent les trous sur la plaque de blocage (6) et entrent à l'intérieur de la poignée (1);
dans lequel le siège de verrouillage comprend: une bride (2-1) ayant un trou traversant, une paroi interne d'un petit cylindre sur le dessus de la bride agencée avec des paliers (2-8) et des éléments de support (2-12) qui supportent les paliers (2-8), la bride étant également pourvue d'un manchon de verrouillage (2-2) qui est agencé avec une première saillie (2-5) sur la paroi interne de sa partie supérieure et une deuxième saillie (2-3) sur la paroi interne de sa partie inférieure ; un ressort (2-9) est agencé entre la deuxième saillie (2-3) et la face de fond supérieure (2-10) de la bride ; une plaque de pression (2-6) est reliée par filetage à une paroi extérieure (2-7) d'une partie de sommet du petit cylindre sur le dessus de la bride et appuie sur la première saillie (2-5) ; des trous de différentes tailles sont prévus sur la paroi interne du petit cylindre correspondant à la deuxième saillie (2-3) et sur les éléments de support (2-12) pour former des trous étagés (2-11) qui sont pourvus de billes d'acier (2-4) à l'intérieur.

2. Trépan crânien selon la revendication 1, **caractérisé en ce que** la face de fond supérieure (2-10) est agencée avec un élément de pression (2-15) relié par filetage à la paroi interne de la face de fond supérieure pour venir en butée étroite contre les paliers. (2-8) et l'élément de support (2-12).

3. Trépan crânien selon la revendication 2, **caractérisé en ce que** les paliers (2-8) sont au nombre de deux qui sont respectivement positionnés en dessous de la paroi externe (2-7) et au-dessus des éléments de pression (2-15).

4. Trépan crânien selon la revendication 3, **caractérisé en ce que** la paroi interne du grand cylindre sur le côté inférieur de la bride (2-1) est pourvue d'un filetage intérieur.

5. Trépan crânien selon la revendication 4, **caractérisé en ce que** tous les fils à l'intérieur du stator du moteur électrique à courant continu ont une couche imperméable à l'eau agencée dessus.

6. Trépan crânien selon la revendication 5, **caractérisé en ce que** l'arbre de sortie du réducteur est pourvu d'une bague d'étanchéité en forme de lèvre.
